# EUROPEAN PATENT APPLICATION

(11) **EP 4 538 258 A1**
(43) Date of publication of application: **16.04.2025**
(21) Application number: 22946079.5
(22) Date of filing: 13.06.2022
(51) Int. Cl.: C07D 233/60, A61K 31/4164, A61P 7/02, A61P 7/10

(54) **IMIDAZOLIDINYL VANILLIC ACID ETHER DERIVATIVE AND USE THEREOF**

(71) Applicant: Hefei Institute of Pharmaceutical Industry Co., Ltd., Hefai, Anhui 230088 (CN); Hefei Amvite Pharmaceutical Co., Ltd., Hefei, Anhui 230601 (CN)
(72) Inventor: HE, Guangwei, Hefei, Anhui 230601 (CN); CHU, Zhaoxing, Hefei, Anhui 230601 (CN); XU, Qinlong, Hefei, Anhui 230601 (CN); LIN, Gaofeng, Hefei, Anhui 230601 (CN); MO, Jiajia, Hefei, Anhui 230601 (CN); ZHAO, Yan, Hefei, Anhui 230601 (CN)
(74) Representative: Hoffmann Eitle
(86) International application number: PCT/CN2022/098306
(87) International publication number: WO 2023/240378

(57) **Abstract**

Provided are an imidazolidinyl vanillic acid ether derivative shown in formula (I) and a use thereof. Compared with the prior art, the imidazolidinyl vanillic acid ether derivative shown in formula (I) has higher in-vivo bioavailability and in-vivo platelet aggregation inhibition activity, and can be used for treating and/or preventing diseases related to thromboembolism.

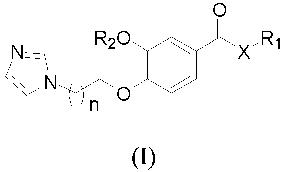

## Description

### Technical Field

The present invention relates to the field of medicinal chemistry, and in particular, to a class of imidazolidinyl vanillic acid ether derivatives and a preparation method thereof. The present invention also provides use of the derivatives in the manufacture of a medicament for treating and/or preventing diseases related to thromboembolic diseases.

### Background Art

Thrombosis caused by various reasons can lead to heart, brain, and lung circulation diseases (e.g., myocardial infarction, stroke, pulmonary embolism, etc.) which are collectively referred to as diseases related to thromboembolism. The incidence of this type of disease ranks among the highest among various diseases, has seriously threatened human life and health, and has become the focus and hot spot of medical research and new medicine development.

Currently, the medicines used in clinical practice to prevent and treat diseases related to thromboembolism mainly include thrombolytic drugs, anticoagulants and platelet aggregation inhibitors. Thrombolytic therapy is mainly used to treat acute ischemic cerebral infarction. However, most patients have missed the opportunity for thrombolysis when they are admitted to the hospital or are unable to undergo thrombolysis due to various conditions, and in addition, thrombolytic drugs have obvious shortcomings, such as being prone to allergies and requiring low-temperature storage, and therefore the clinical application of this type of drug is greatly limited. Anticoagulants have definite therapeutic effects, but their effects are limited to venous thrombosis, and in addition, the risk of bleeding is relatively high, and therefore anticoagulants are not as widely used as platelet aggregation inhibitors. Platelet aggregation inhibitors are the most commonly used medicines for arterial thrombosis, and the representative includes the cyclooxygenase inhibitor aspirin, the P₂Y₁₂ receptor inhibitor clopidogrel, ticagrelor, etc. P₂Y₁₂ receptor inhibitors have become the main medicines used in clinical practice. However, some patients still have problems such as insufficient efficacy or bleeding risks (Zhang Xia, Ke Yongsheng, Progress in clinical research on the new P2Y12 receptor inhibitor ticagrelor [J], Chinese Journal of Clinical Pharmacology and Therapeutics, 2014, 19(4): 459-465). There is still a strong demand for new platelet aggregation inhibitors.

Chinese invention patent CN101851209B discloses a carboxylic acid compound (Compound A) of the following formula having platelet aggregation inhibition activity, and discloses a methyl ester intermediate of the compound in its synthesis route. It was found that this compound had a significant inhibitory effect on adenosine diphosphate (ADP)-induced platelet aggregation in rabbit (there was no activity data with regard to its methyl ester intermediate). However, this compound has low oral bioavailability and weak ability to penetrate the blood-brain barrier, which limits its clinical application.

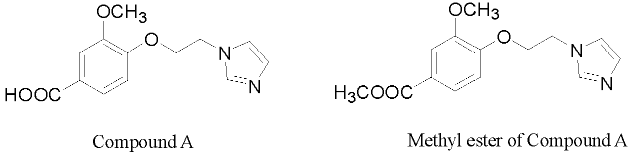

There is still requirement for medicines with stronger antiplatelet aggregation activity and higher bioavailability in the prior art.

### Summary of the Invention

In order to solve the above problems existing in the prior art, the inventors of the present invention have designed a class of imidazolidinyl vanillic acid derivatives having a structure shown in the following formula (I): 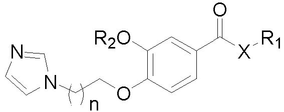 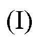 or a pharmaceutically acceptable salt, stereoisomer, tautomer, prodrug, or solvate thereof, wherein:
X is selected from O or NH;
R₁ is selected from C₃ - C₁₂ alkyl, C₃ - C₁₂ alkenyl, C₃ - C₁₂ alkynyl, C₃ - C₁₂ cycloalkyl, or C₆ - C₁₄ aryl, wherein the alkyl, alkenyl, alkynyl, and cycloalkyl are optionally substituted by halogen, cyano, C₁ - C₁₂ alkoxy, C₁ - C₁₂ alkoxycarbonyl, C₁ - C₁₂ alkoxycarbonyloxy, C₁ - C₁₂ alkylacyloxy, carboxyl, C₁ - C₁₂ alkylthio, C₁ - C₁₂ alkylthiocarbonyl, C₁ - C₁₂ alkylthiocarbonyloxy, C₁ - C₁₂ alkylthiocarbonylthio, C₁ - C₁₂ alkylacylthio, C₁ - C₁₂ alkylamino, C₁ - C₁₂ alkylaminocarbonyl, C₁ - C₁₂ alkylaminoacyloxy, C₁ - C₁₂ alkylamide, or C₁ - C₁₂ alkylaminoamide; the aryl is optionally substituted by C₁ - C₁₂ alkyl, C₁ - C₁₂ alkoxy, OH, halogen, cyano, nitro, or carboxyl, wherein the alkyl and the alkoxy are optionally substituted by OH, amino, carboxyl, nitro, cyano, halogen, C₁ - C₁₂ alkoxy, or C₃ - C₁₂ cycloalkyl ; or R₁ is selected from C₁ - C₂ alkyl substituted by C₁ - C₁₂ alkoxycarbonyloxy or C₁ - C₁₂ alkylacyloxy, or benzyl substituted by hydroxyl;
R₂ is selected from H or C₁ - C₁₂ alkyl; and
n is selected from 0, 1, 2, or 3.
In one embodiment, R₁ is selected from C₃ - C₁₂ alkyl, C₃ - C₁₂ cycloalkyl, or C₆ - C₁₄ aryl, wherein the alkyl and cycloalkyl are optionally substituted by halogen, cyano, C₁ - C₁₂ alkoxy, C₁ - C₁₂ alkoxycarbonyl, C₁ - C₁₂ alkoxycarbonyloxy, C₁ - C₁₂ alkylacyloxy, carboxyl, C₁ - C₁₂ alkylthio, C₁ - C₁₂ alkylthiocarbonyl, C₁ - C₁₂ alkylthiocarbonyloxy, C₁ - C₁₂ alkylthiocarbonylthio, C₁ - C₁₂ alkylacylthio, C₁ - C₁₂ alkylamino, C₁ - C₁₂ alkylaminocarbonyl, C₁ - C₁₂ alkylaminoacyloxy, C₁ - C₁₂ alkylamide, or C₁ - C₁₂ alkylaminoamide; the aryl is optionally substituted by C₁ - C₁₂ alkyl, C₁ - C₁₂ alkoxy, OH, halogen, cyano, nitro, or carboxyl, wherein the alkyl and the alkoxy are optionally substituted by OH, amino, carboxyl, nitro, cyano, halogen, C₁ - C₁₂ alkoxy, or C₃ - C₁₂ cycloalkyl ; or R₁ is selected from C₁ - C₂ alkyl substituted by C₁ - C₁₂ alkoxycarbonyloxy or C₁ - C₁₂ alkylacyloxy, or benzyl substituted by hydroxyl.

In one embodiment, R₁ is selected from C₃ - C₁₂ alkyl, C₃ - C₁₂ cycloalkyl, or C₆ - C₁₄ aryl, wherein the alkyl and cycloalkyl are optionally substituted by halogen, C₁ - C₁₂ alkoxy, C₁ - C₁₂ alkoxycarbonyl, C₁ - C₁₂ alkoxycarbonyloxy, C₁ - C₁₂ alkylacyloxy, or carboxyl; the aryl is optionally substituted by C₁ - C₁₂ alkyl, C₁ - C₁₂ alkoxy, or OH, wherein the alkyl and alkoxy are optionally substituted by OH, amino, or carboxyl; or R₁ is selected from C₁ - C₂ alkyl substituted by C₁ - C₁₂ alkoxycarbonyloxy or C₁ - C₁₂ alkylacyloxy , or benzyl substituted by hydroxyl.

In one embodiment, R₁ is selected from C₃ - C₆ alkyl, C₃ - C₈ cycloalkyl, or phenyl, wherein the alkyl and cycloalkyl are optionally substituted by halogen, C₁ - C₆ alkoxy, C₁ - C₆ alkoxycarbonyl, C₁ -C₆ alkoxycarbonyloxy, C₁ -C₆ alkylacyloxy, or carboxyl; the phenyl is optionally substituted by C₁ - C₆ alkyl, C₁ - C₆ alkoxy, or OH, wherein the alkyl and alkoxy are optionally substituted by amino or carboxyl; or R₁ is selected from C₁ - C₂ alkyl substituted by C₁ - C₆ alkoxycarbonyloxy or C₁ - C₆ alkylacyloxy, or benzyl substituted by hydroxyl.

In one embodiment, R₁ is selected from: n-propyl, isopropyl,

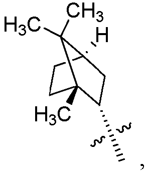

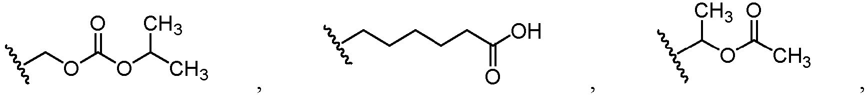

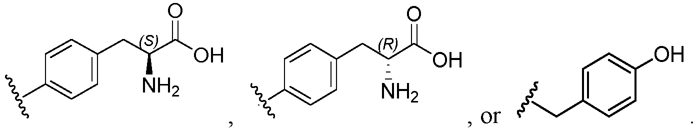

In one embodiment, R₂ is selected from H or C₁ - C₆ alkyl; preferably, R₂ is selected from H or methyl.

In one embodiment, n is selected from 0 or 1; preferably, n is selected from 1.

In one embodiment, the compound of the present invention is selected from: 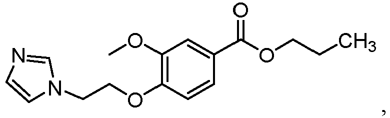 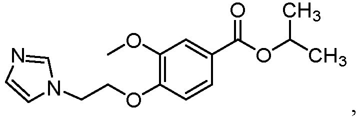 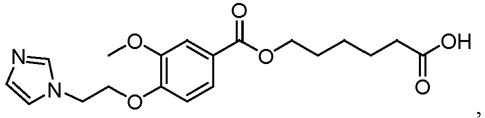 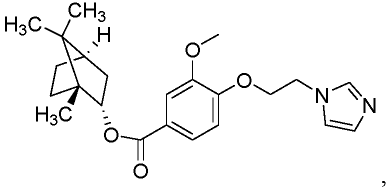 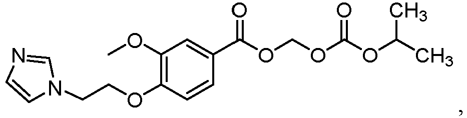 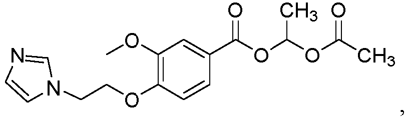 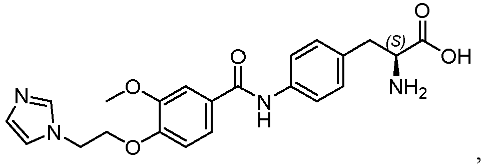 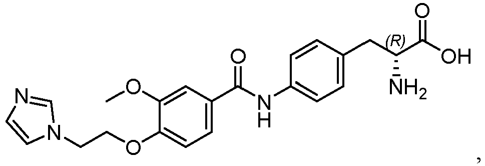 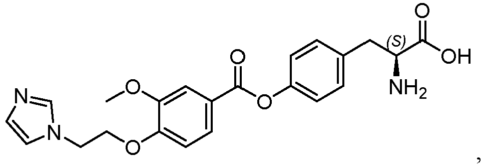 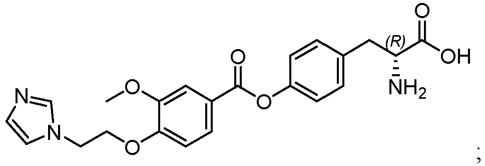 or a pharmaceutically acceptable salt, stereoisomer, tautomer, prodrug, or solvate thereof.

The present invention also provides a pharmaceutical composition for treating and/or preventing diseases related to thromboembolism, including a compound having a structure represented by formula (I) or a pharmaceutically acceptable salt, stereoisomer, tautomer, prodrug, or solvate thereof, and a pharmaceutically acceptable carrier.

The present invention also provides the use of a compound having a structure represented by formula (I) or a pharmaceutically acceptable salt, stereoisomer, tautomer, prodrug, or solvate thereof in the manufacture of a medicament for treating and/or preventing diseases related to thromboembolism.

The present invention also provides a method for treating and/or preventing diseases related to thromboembolism, including administration of a therapeutically effective amount of a compound having a structure represented by formula (I) or a pharmaceutically acceptable salt, stereoisomer, tautomer, prodrug, or solvate thereof to an individual in need thereof.

The pharmaceutical composition including the compound of the present invention, the use of the compound of the present invention, or the method of treating or preventing diseases related to thromboembolism using the compound of the present invention as described above are provided, wherein diseases related to thromboembolism are diseases caused by platelet aggregation.

The compound of the present invention, or pharmaceutically acceptable salt, stereoisomer, tautomer, prodrug, and solvate thereof having stronger antiplatelet aggregation activity in vivo and higher bioavailability, may meet the clinical requirement of various thromboembolic diseases, and may maximize the clinical treatment advantages and application scope.

### Detailed Description of the Invention

### Definition

As used in this specification, the following words and phrases are generally intended to have the meanings set forth below, unless the context in which they are used indicates otherwise.

As used herein, the term "alkyl" refers to a monovalent group of a branched or unbranched saturated hydrocarbon chain having 1 to 12 carbon atoms, more typically 1 to 10 carbon atoms, 1 to 8 carbon atoms, or 1 to 6 carbon atoms. This term is exemplified by groups such as methyl, ethyl, 1-propyl (n-propyl), 2-propyl (isopropyl), 1-butyl (n-butyl), 2-methyl-1-propyl (isobutyl), 2-butyl (sec-butyl), 2-methyl-2-propyl (tert-butyl), 1-pentyl (n-pentyl), 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, 1-heptyl, 1-octyl, 1-nonyl, 1-decyl, and the like.

As used herein, the term "alkenyl" refers to a monovalent unsaturated straight or branched hydrocarbon radical with the indicated number of carbon atoms (e.g. with 3 to 12 carbon atoms, 2 to 10 carbon atoms, 2 to 8 carbon atoms, or 2 to 6 carbon atoms, etc.) and carbon-carbon double bond (s) (e.g. one, two, or three carbon-carbon double bonds). In some embodiments, examples of alkenyl groups include, but are not limited to, ethenyl (i.e., -CH=CH₂), propen-1-yl (i.e., -CH=CHCH₃), propen-3-yl (or allyl, i.e., -CH₂CH=CH₂), propen-2-yl (i.e., -C(CH₃)=CH₂), butadienyl (including 1,2-butadienyl and 1,3-butadienyl), and the like.

As used herein, the term "alkynyl" refers to a monovalent unsaturated straight or branched hydrocarbon radical with the indicated number of carbon atoms (e.g. with 3 to 12 carbon atoms, 2 to 10 carbon atoms, 2 to 8 carbon atoms, or 2 to 6 carbon atoms, etc.) and carbon-carbon triple bond (s) (e.g., one, two, or three carbon-carbon triple bonds). In some embodiments, examples of alkynyl groups include, but are not limited to, ethynyl (i.e., -C≡CH), propargyl (i.e., -CH₂C≡CH), propynyl (i.e., -C≡CCH₃), etc.

As used herein, the term "cycloalkyl" refers to a monovalent saturated carbocyclic group of 3 to 12 carbon atoms, more typically 3 to 10 carbon atoms, or 3 to 8 carbon atoms, having a single ring or multiple fused or bridged rings. In some embodiments, the cycloalkyl includes a monocyclic structure (e.g., cyclopropyl, cyclobutyl, cyclopentyl, cyclooctyl, etc), or a polycyclic structure (e.g., adamantyl and bicyclo[2.2.1]heptanyl), or a cycloalkyl group fused to an aryl group (e.g., indane, etc), if the point of attachment is through the cycloalkyl group.

As used herein, the term "aryl" refers to an aromatic carbocyclic group of 6 to 14 carbon atoms (more typically 6 to 10 carbon atoms, or 6 carbon atoms) having a single ring (e.g., phenyl) or multiple rings (e.g., biphenyl), or multiple condensed (fused) rings (e.g., naphthyl, fluorenyl, and anthryl). Exemplary groups of this term include phenyl, fluorenyl, naphthyl, anthryl, 1,2,3,4-tetrahydronaphthalene (if the point of attachment is through an aryl group), and the like.

As used herein, the term "halogen" refers to fluorine, chlorine, bromine and iodine.

As used herein, the term "alkoxy" refers to an "alkyl-O-" group, wherein alkyl is as defined herein. This term is exemplified by groups such as methoxy, ethoxy, n-propoxy, isopropoxy, n-butoxy, isobutoxy, tert-butoxy, and the like.

As used herein, the term "therapeutically effective amount" refers to an amount that is sufficient to affect treatment, as defined below, when administered to a mammal in need of such treatment. The therapeutically effective amount will vary depending upon the subject and disease condition being treated, the weight and age of the subject, the severity of the disease condition, the manner of administration and the like, which can readily be determined by one of ordinary skill in the art.

As used herein, the term "stereoisomer" refers to compounds that have identical chemical constitution and connectivity but have different orientations of their atoms in space that are not interchangeable by rotation about single bonds. "Stereoisomers" include "diastereomers" and "enantiomers". "Diastereomers" refers to stereoisomers that have two or more chiral centers and whose molecules are not mirror images of one another. Diastereomers have different physical properties, e.g., melting points, boiling points, spectral characteristics, and reactivity. Diastereomeric mixtures may be separated under high resolution analytical procedures such as crystallization, electrophoresis and chromatography. "Enantiomers" refers to two stereoisomers of a compound that are non-superimposable mirror images of one another.

As used herein, the term "tautomer" refers to the coexistence of two (or more) compounds that differ only in the position and electron distribution of one (or more) mobile atoms, such as keto-enol tautomers.

As used herein, the term "pharmaceutically acceptable salt" refers to salts that retain the biological effectiveness and properties of the given compound, and which are not biologically or otherwise undesirable. Pharmaceutically acceptable salts can be prepared from inorganic and organic acids. Salts derived from inorganic acids include hydrochlorides, hydrobromides, sulfates, nitrates, phosphates, carbonates, bisulfates, hydrophosphates, dihydrophosphates, bicarbonates, and the like. Salts derived from organic acids include formates, acetates, propionates, glycolates, pyruvates, oxalates, malates, malonates, succinates, maleates, fumarates, tartrates, citrates, benzoates, cinnamates, mandelates, methanesulfonates, ethanesulfonates, p-toluenesulfonates, salicylates, and the like.

As used herein, the term "pharmaceutically acceptable" means that the substance or composition must be chemically and/or toxicologically compatible with the other ingredients of those comprising the formulation and/or the mammal to be treated therewith.

As used herein, a "prodrug" is a compound that, upon in vivo administration, is metabolized by one or more steps or processes or otherwise converted to the biologically, pharmaceutically or therapeutically active form of the compound. To produce a prodrug, the pharmaceutically active compound is modified such that the active compound may be regenerated by metabolic processes. The prodrug may be designed to alter the metabolic stability or the transport characteristics of a drug, to mask side effects or toxicity, to improve the flavor of a drug or to alter other characteristics or properties of a drug. By virtue of knowledge of pharmacodynamic processes and drug metabolism in vivo, those of ordinary skill in the art, once a pharmaceutically active compound is known, can design prodrugs of the compound (see, e.g., Nogrady (1985) Medicinal Chemistry A Biochemical Approach, Oxford University Press, New York, pages 388-392).

As used herein, the term "solvate" refers to an association compound or complex of one or more solvent molecules and the compound of the present invention. Examples of solvents that form solvates include, but are not limited to, water, isopropanol, ethanol, methanol, DMSO, ethyl acetate, acetic acid, and ethanolamine. The term "hydrate" refers to a complex wherein the solvent molecule is water.

### Pharmaceutical compositions and administration

The compounds provided by the present invention or their pharmaceutically acceptable salts, stereoisomers, tautomers, prodrugs, solvates are usually administered in the form of pharmaceutical compositions. The present invention therefore provides a pharmaceutical composition comprising a compound provided by the present invention as an active ingredient and one or more pharmaceutically acceptable carriers. The pharmaceutical composition can be administered alone or in combination with other therapeutic agents. Such compositions are prepared in a manner well known in the art (eg, Reminton's Pharmaceutical Sciences, Mace Publishing Co., Philadelphia, PA 17th Ed. (1985); and Modern Pharmaceutics, Marcel Dekker, Inc. 3rd Ed. (GS Banker & CT Rhodes, Eds.).

Pharmaceutically acceptable carriers can be solid or liquid. Among them, the solid carrier may be one or more materials used as excipients, diluents, sweeteners, solubilizers, lubricants, binders, tablet disintegrants, stabilizers, preservatives, or encapsulating materials. The liquid carrier may be solvents or liquid dispersion media. Suitable solid carriers include, but are not limited to, for example, cellulose, glucose, lactose, mannitol, magnesium stearate, magnesium carbonate, sodium carbonate, saccharin sodium, sucrose, dextrin, talc, starch, pectin, gelatin, tragacanth, arabic gum, sodium alginate, parabens, methylcellulose, sodium carboxymethyl cellulose, a lowmelting point wax, cocoa butter, and the like. Suitable liquid carriers include, but are not limited to, water, ethanol, polylol (such as glycerol, propanediol, liquid polyethylene glycol, etc), a vegetable oil (such as peanut oil, cottonseed oil, safflower oil, sesame oil, olive oil, corn oil, and soybean oil), glyceride, agar, pyrogen-free water, isotonic saline, Ringer's solution, and a mixture thereof.

The pharmaceutical compositions according to the present invention may be in any form suitable for the intended method of administration. For example, when used for oral use, it can be prepared into tablets, lozenges, buccal tablets, aqueous or oily suspensions, dispersible powders or granules, emulsions, hard or soft capsules, syrups or elixirs, solutions, sprays. Compositions intended for oral use may be prepared according to any method known in the art for the manufacture of pharmaceutical compositions. The pharmaceutical composition of the present invention may be in the form of a sterile injection preparation, such as a sterile injection aqueous or oily suspension. The pharmaceutical composition of the present invention can also be prepared into a preparation suitable for intrapulmonary or intranasal administration, such as a preparation for aerosol or dry powder administration, nasal drops or nasal sprays. The pharmaceutical composition of the present invention can also be prepared into suppositories suitable for rectal administration. The pharmaceutical composition of the present invention can also be prepared into a transdermal preparation for topical administration or an eye drop suitable for ocular administration.

The pharmaceutical composition of the present invention can be administered via arterial injection, intravenous injection, intraperitoneal injection, parenteral administration, intramuscular administration, subcutaneous administration, oral administration, topical administration in single doses or multiple doses.

The effective dosage of the compounds of the present invention will depend at least on the nature of the condition being treated, toxicity, method of delivery, and pharmaceutical formulation, and will be determined by the clinician using routine dose escalation studies. About 0.0001 to about 100 mg/kg body weight per day can be expected; typically about 0.01 to about 10 mg/kg body weight per day; more typically about 0.01 to about 5 mg/kg body weight per day; most typically about 0.05 to about 0.5 mg/kg body weight per day. For example, a candidate daily dosage for an adult human of about 70 kg body weight would be in the range of 1 mg to 1000 mg, preferably in the range of 5 mg to 500 mg, and may be in the form of a single dose or multiple doses.

### Indications

The compounds of the present invention or a pharmaceutically acceptable salts, stereoisomers, tautomers, prodrugs, or solvates thereof can be used for preventing and/or treating thromboembolic diseases. In particular, the compounds of the present invention or their pharmaceutically acceptable salts, stereoisomers, tautomers, prodrugs, solvates can be used to treat and/or prevent atherosclerosis, coronary heart disease, myocardial infarction, ischemic stroke, peripheral vascular disease, multiple sclerosis, scleroderma, Raynaud's phenomenon caused by multiple sclerosis, deep vein thrombosis, lower extremity vein thrombosis, intermittent claudication, etc.

### Combination medication

The compounds of the present invention can be administered as a single therapeutic agent to treat and/or prevent thromboembolic diseases, or other diseases caused by platelet aggregation, or diseases selected from atherosclerosis, coronary heart disease, myocardial infarction, ischemic stroke, peripheral vascular disease, multiple sclerosis, scleroderma, Raynaud's phenomenon caused by multiple sclerosis, deep vein thrombosis, lower extremity venous thrombosis, intermittent claudication, etc. The compounds of the present invention may also be administered in combination with one or more additional therapeutic agents to treat the diseases mentioned herein. The additional therapeutic agent(s) may be any antithrombotic agent with the same or different mechanism of action, for example: the anticoagulant unfractionated heparin, low molecular weight heparin, other heparin derivatives, synthetic heparin derivatives (such as fondaparinheparin (fondaparinux), vitamin K antagonists, synthetic or biotechnological inhibitors of nonthrombin coagulation factors (such as synthetic FXa, FVIIa, and FIXa inhibitors and rNAPc2), antiplatelet agents (acetylsalicylic acid, ticlopidine, and clopidogrel); thromboxane receptor and/or synthetase inhibitors; fibrinogen receptor antagonists; pseudo-prostacyclins; phosphodiesterase inhibitors; ADP-receptor (P2X1, P2Y1, P2Y12 [P2T]) antagonists; and carboxylic peptidase U inhibitors (CPU or TAFIa) and inhibitors of plasminogen activator inhibitor-1 (PAI-1).

The compounds of the present invention can also be administered in combination with thrombolytic drugs to treat and/or prevent thromboembolic diseases, or diseases caused by platelet aggregation, or diseases selected from atherosclerosis, coronary heart disease, myocardial infarction, ischemic stroke, peripheral vascular disease, multiple sclerosis, scleroderma, Raynaud's phenomenon caused by multiple sclerosis, deep vein thrombosis, lower extremity venous thrombosis, intermittent claudication, etc. Thrombolytic drugs include, for example, one or more tissue plasminogen activators (natural, recombinant or modified), streptokinase, urokinase, pro-urokinase, anisoylated plasminogen streptokinase activator complex (APSAC), animal salivary gland plasminogen activator, etc.

### Treatment methods and uses

In one embodiment, the present invention provides a method for treating and/or preventing thromboembolic diseases, the method comprising administering to a subject in need thereof a therapeutically effective amount of a compound of the present invention or a pharmaceutically acceptable salt, stereoisomer, tautomer, prodrug, solvate thereof, or a pharmaceutical composition comprising the same. In another embodiment, the present invention provides a method for treating and/or preventing diseases caused by platelet aggregation, the method comprising administering to an individual in need thereof a therapeutically effective amount of a compound of the present invention or a pharmaceutically acceptable salt, stereoisomer, tautomer, prodrug, solvate, or a pharmaceutical composition comprising the same. In another embodiment, the present invention provides a method for treating and/or preventing atherosclerosis, coronary heart disease, myocardial infarction, ischemic stroke, peripheral vascular disease, multiple sclerosis, scleroderma, Raynaud's phenomenon caused by multiple sclerosis, deep vein thrombosis, lower extremity vein thrombosis, and/or intermittent claudication, the method comprising administering to an individual in need thereof a therapeutically effective amount of a compound of the present invention or a pharmaceutically acceptable salt, stereoisomer, tautomer, prodrug, solvate thereof, or a pharmaceutical composition comprising the same.

The present invention provides use of the compounds of the present invention or their pharmaceutically acceptable salt, stereoisomer, tautomer, prodrug, solvate as active therapeutic substances in the manufacture of a medicament for treating and/or preventing thromboembolic diseases. More particularly, the present invention provides the use of the compounds of the present invention or their pharmaceutically acceptable salts, stereoisomers, tautomers, prodrugs, solvates as active therapeutic substances in the manufacture of a medicament for treating and/or preventing diseases caused by platelet aggregation. More particularly, the present invention provides the use of the compounds of the present invention or their pharmaceutically acceptable salts, stereoisomers, tautomers, prodrugs, solvates as active therapeutic substances in the manufacture of a medicament for treating and/or preventing the following diseases, including atherosclerosis, coronary heart disease, myocardial infarction, ischemic stroke, peripheral vascular disease, multiple sclerosis, scleroderma, Raynaud's phenomenon caused by multiple sclerosis, deep vein thrombosis, lower extremity vein thrombosis, and/or intermittent claudication.

### General synthetic method

The compounds of the present invention can be prepared using the methods disclosed herein and modifications thereof as well as methods well known in the art. Typical embodiments of the compounds according to the present invention can be synthesized using the following general reaction schemes. It is apparent from the description herein that correspondingly different products can be obtained by replacing the reaction starting materials with other materials having similar structures. Reaction starting materials were typically obtained from commercial sources or synthesized using published methods.

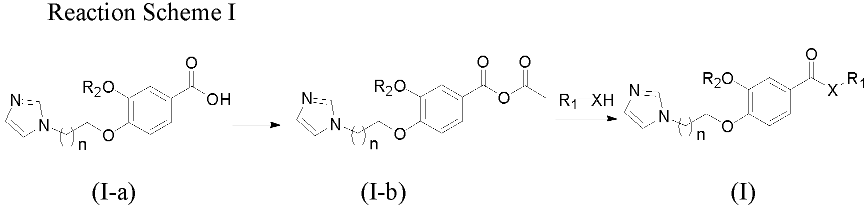

The carboxylic acid compound represented by formula (I-a) is reacted with an acid anhydride compound (such as acetic anhydride, trifluoroacetic anhydride, etc.) or an acyl halide compound (such as acetyl chloride, acetyl bromide, etc.) in an inert solvent to generate a mixed acid anhydride compound (such as formula (I-b)), which is then reacted with compound R₁XH to obtain the compound represented by formula (I).

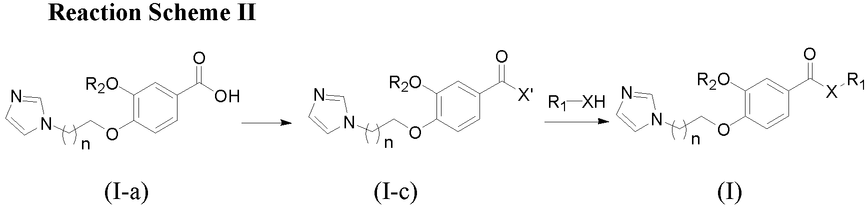

The carboxylic acid compound represented by formula (I-a) is reacted with a halogenating agent (such as oxalyl chloride, sulfinyl chloride, etc.) in an inert solvent to generate an acyl halide (such as the compound represented by formula (I-c), wherein X' is chlorine or bromine), which is reacted with R₁XH to obtain the compound represented by formula (I).

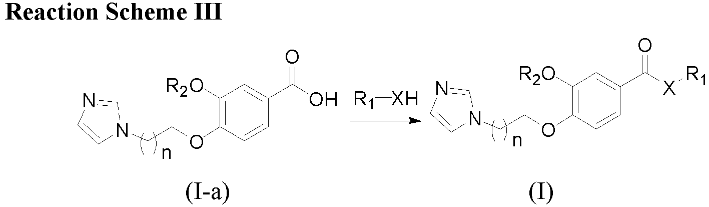

The compound represented by formula (I-a) is reacted with a compound represented by formula R₁XH under conditions suitable for forming an amide or an ester to obtain the compound represented by formula (I). For example, a condensing agent (such as 2-(7-aza-1H-benzotriazol-1-yl)-1,1,3,3-tetramethyluronium hexafluorophosphate (HATU), dicyclohexylcarbodiimide (DCC), diisopropylcarbodiimide (DIC), 1-(3-dimethylaminopropyl)-3-ethylcarbodiimide (EDCI) and 1-hydroxybenzotriazole (HOBt), etc.) and a base (such as N-methylmorpholine, 4-dimethylaminopyridine, triethylamine, diisopropylethylamine, etc.) are added to a mixture of the compound represented by formula (I-a) and R₁XH in an inert solvent to react and obtain the compound represented by formula (I).

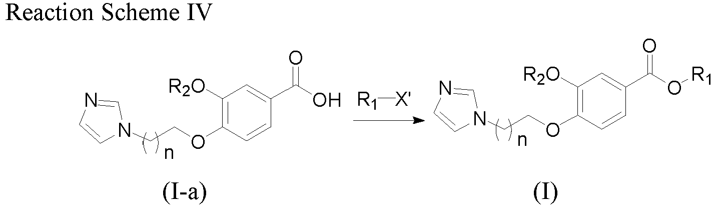

The carboxylic acid represented by formula (I-a) is reacted with R₁X' in an inert solvent under an alkaline condition (e.g., cesium carbonate, potassium carbonate, etc.) to obtain the compound represented by formula (I).

The following examples are provided to illustrate the preparation of compounds of the present invention, but are not intended to limit the invention in any way.

### Examples

### Example 1 Synthesis of Compound propyl 4-(2-(1H-imidazol-1-yl)ethoxy)-3-methoxybenzoate

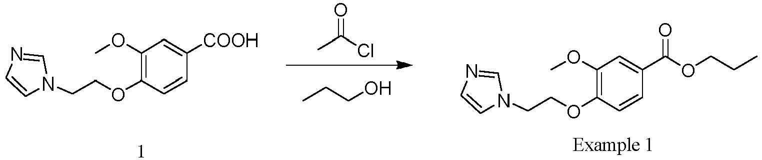

Compound 1 (i.e., compound A mentioned in the background art) (2.0 g, 7.63 mmol), n-propanol (30 mL) and acetyl chloride (1.2 g, 15.3 mmol) were added to a 100 mL single-necked flask and reacted at 100 °C for 48 h. Then, the reaction solution was concentrated under reduced pressure and purified by column chromatography (*V*_{dichloromethane}:*V*ₘₑₜₕₐₙₒₗ = 50:1-10:1, alkalized with triethylamine) to obtain 2.1 g of Example 1 compound as a white solid in a yield of 90.5%.

¹H NMR (400 MHz, CDCl₃) *δ* (ppm): 7.70-7.61 (m, 2H, ArH), 7.58 (d, *J*=1.71 Hz, 1H, ArH), 7.10 (d, J=11.49 Hz, 2H, ArH), 6.80 (d, J=8.56 Hz, 1H, ArH), 4.39-4.44 (m, 2H, CH₂), 4.24-4.34 (m, 4H, CH₂CH₂), 3.93 (s, 3H, OCH₃), 1.74-1.85 (m, 2H, CH₂), 1.04 (t, J=7.34 Hz, 3 H, CH₃).

### Example 2 Synthesis of Compound isopropyl 4-(2-(1H-imidazol-1-yl)ethoxy)-3-methoxybenzoate hydrochloride

### Step 1

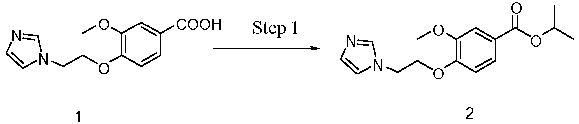

Compound 1 (2.0 g, 7.63 mmol), isopropanol (45 mL) and acetyl chloride (1.2 g, 15.3 mmol) were added to a 100 mL single-necked flask. The mixture was heated to 90°C under nitrogen atmosphere and reacted overnight. TLC (*V*_{dichloromethane}:*V*ₘₑₜₕₐₙₒₗ = 20: 1) detected that the reaction conversion was about 50%. The reaction solution was cooled to room temperature, and dichloromethane (40 mL) was added and stirred for 10 minutes. The mixture was then filtered and the filtrate was concentrated under reduced pressure. It was purified by column chromatography (*V*_{dichloromethane}:*V*ₘₑₜₕₐₙₒₗ = 50:1-10:1, alkalized with triethylamine) to obtain 1.02 g of intermediate 2 as a colorless oil with a yield of 44.0%.

### Step 2

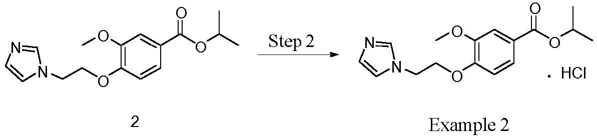

Intermediate 2 (100 mg, 0.33 mmol), ethyl acetate (1.5 mL) and HCl in ethyl acetate (4 mL, 3 N) were added into a 25 mL single-necked flask. The reaction was stirred overnight under nitrogen atmosphere. The reaction solution was then heated to 50°C and reacted for another 3 h. The reaction solution was concentrated under reduced pressure to obtain 130 mg of Example 2 compound as a white solid with a yield of 44.0%. ¹H NMR (400 MHz, DMSO-*d*₆) *δ* (ppm):9.20 (br, 1H, ArH), 7.82 (br, 1H, ArH), 7.67 (br, 1H, ArH), 7.54 (d, *J*=8.06 Hz, 1H, ArH), 7.44 (s, 1H, ArH), 7.12 (d, *J*=8.31 Hz, 1 H, ArH), 5.03-5.17 (m, 1H, CH), 4.67 (br, 2H, CH₂), 4.48 (br, 2H, CH₂), 3.81 (s, 3H, CH₃), 1.31 (d, *J*=6.04 Hz, 6H, CH₃CH₃).

### Example 3 Synthesis of Compound 6-(4-(2-(1H -imidazol-1-yl)ethoxy)-3-methoxybenzoyloxy)hexanoic acid

### Step 1

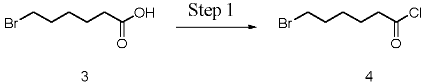

Compound 3 (5.00 g, 25.6 mmol), dichloromethane (25 mL) and thionyl chloride (6.10 g, 51.3 mmol) were added sequentially into a 100 mL single-necked flask and reacted at 50 °C for 1 h under nitrogen atmosphere. After the reaction was completed as monitored with TLC (*V*_{petroleum ether}:*V*_{ethyl acetate}= 2:1), the reaction solution was concentrated under reduced pressure and evaporated with toluene (5 mL × 3) to obtain 5.47 g of yellow oily intermediate 4, which was used directly in the next step without purification.

### Step 2

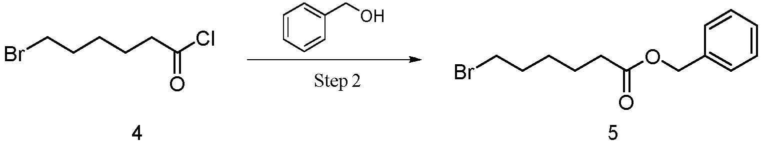

Benzyl alcohol (2.6 mL, 25.0 mmol), dichloromethane (25 mL), triethylamine (7.1 mL, 51.3 mmol) and 4-dimethylaminopyridine (DMAP) (285 mg, 2.33 mmol) were added to a 100 mL single-necked flask in sequence, a solution of intermediate 4 (5.47 g, 25.6 mmol) in dichloromethane (10 mL) was added dropwise at 0 °C with stirring, and the reaction was carried out at room temperature for 3 h under nitrogen atmosphere. After the reaction was completed as monitored with TLC (*V*_{petroleum ether}:*V*_{ethyl acetate}=10:1), the reaction system was diluted with dichloromethane (45 mL), washed with saturated brine (10 mL × 3), and the organic phase was collected. The mixture was dried over anhydrous sodium sulfate, filtered, concentrated and purified by column chromatography (*V*_{petroleum ether}: *V*_{ethyl acetate}= 20:1) to obtain 6.65 g of intermediate 5 as a colorless oil with a yield of 90.9%.

### Step 3

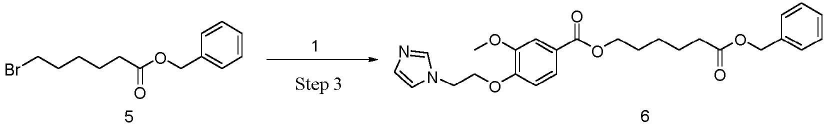

Compound 1 (1.00 g, 3.81 mmol), N,N-dimethylformamide (DMF) (10 mL) and sodium bicarbonate (0.80 g, 9.53 mmol) were added to a 50 mL single-necked flask and reacted at 75 °C for 1 h. To the above reaction solution, a solution of intermediate 5 (1.30 g, 4.58 mmol) in DMF (2 mL) was added dropwise and the mixture was reacted at 75 °C overnight. After the reaction was completed by monitoring with TLC (*V*_{dichloromethane}:*V*ₘₑₜₕₐₙₒₗ=10:1), the reaction system was quenched with water (12 mL), and extracted with ethyl acetate (10 mL × 6), the organic phases were combined, washed with saturated brine (5 mL × 6), and the organic phase was collected. The mixture was dried over anhydrous sodium sulfate, filtered, concentrated and purified by column chromatography (*V*_{dichloromethane}:*V*ₘₑₜₕₐₙₒₗ= 10:1) to obtain 220 mg of intermediate 6 as a colorless oil with a yield of 10.3%.

### Step 4

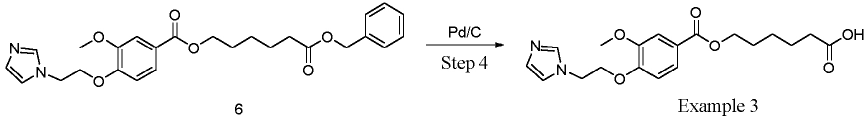

Intermediate 6 (210 mg, 0.45 mmol) and tetrahydrofuran (5 mL) were added to a 25 mL single-necked flask and stirred at room temperature until completely dissolved. 10% Pd/C (42 mg, 20% wt) was added under nitrogen atmosphere which was then replaced with hydrogen for three times, and the reaction was carried out at room temperature for 2.5 h. After the reaction was completed as monitored with TLC (*V* _{dichloromethane:}*V* ₘₑₜₕₐₙₒₗ = 10: 1), the mixture was filtered through celite pad and the filtrate was concentrated to obtain a crude product. Ethyl acetate (5 mL) was added and the mixture was stirred to precipitate a large amount of solid, which was filtered and the filter cake was oven dried to obtain 110 mg of Example 3 compound as a white solid with a yield of 65.1%. ¹H NMR (400 MHz, CDCl₃) *δ* (ppm): 8.56 (s, 1H, ArH), 7.70 (s, 1H, ArH), 7.53-7.51 (m, 1H, ArH), 7.46 (d, *J*=1.6Hz, 1H, ArH), 7.01 (d, *J*=4.0Hz, 2H, ArH), 6.68 (d, *J*=8.4Hz, 1H, ArH), 4.31-4.29 (m, 2H, CH₂), 4.24-4.21 (m, 4H, 2CH₂), 3.82 (s, 3H, CH₃), 2.29 (t, *J*=7.6Hz, 2H, CH₂), 1.75-1.61 (m, 4H, 2CH₂), 1.47-1.40 (m, 2H, CH₂).

### Example 4 Synthesis of Compound (1R,4R)-4,7,7-trimethylbicyclo[2.2.1]heptan-2-yl-4-(2-(1H-imidazol-1-yl)ethoxy)-3-methoxybenzoate

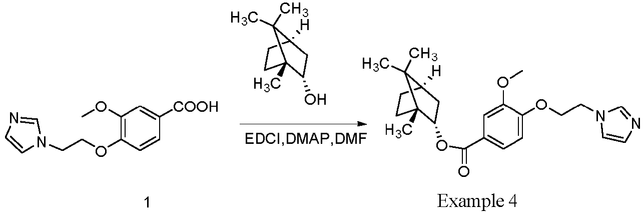

Compound 1 (1.31 g, 5 mmol), borneol (0.77 g, 5 mmol), EDCI (1.15 g, 6 mmol), DMAP (0.73 g, 6 mmol) and DMF (60 mL) were added to a 100 mL single-necked flask and stirred at room temperature for 16 h under nitrogen atmosphere. After the reaction was completed as monitored with TLC (*V*_{dichloromethane}:*V*ₘₑₜₕₐₙₒₗ= 20:1), water (40 mL) was added to the reaction solution, and the mixture was extracted with ethyl acetate (40 mL×3). The organic phases were combined, washed with saturated brine (40 mL×3), dried over anhydrous sodium sulfate, filtered, concentrated, and purified by column chromatography (*V*_{dichloromethane}:*V*ₘₑₜₕₐₙₒₗ= 20:1) to obtain 1.52 g of Example 4 compound as a white solid with a yield of 76.3%. ¹H NMR (400 MHz, DMSO- *d*₆) *δ* (ppm): 7.70 (s, 1H, ArH), 7.60 (dd, *J*=8.4 Hz, *J*=2.0 Hz, 1H, ArH), 7.48 (d, *J*=2.0 Hz, 1H, ArH), 7.26 (s, 1H, ArH), 7.10 (d, *J*=8.4 Hz, 1H, ArH), 6.91 (s, 1H, ArH), 5.03-4.99 (m, 1H, CH), 4.42-4.39 (m, 2H, NCH₂), 4.35-4.32 (m, 2H, OCH₂), 3.83 (s, 3H, OCH₃), 2.42-2.34 (m, 1H, CH₂), 2.09-2.02 (m, 1H, CH₂), 1.80-1.71 (m, 2H, CH₂), 1.43-1.35 (m, 1H, CH₂), 1.32-1.26 (m, 1H, CH₂), 1.08-1.04 (m, 1H, CH₂), 0.93 (s, 3H, CH₃), 0.90 (s, 3H, CH₃), 0.86 (s, 3H, CH₃).

### Example 5 Synthesis of Compound ((isopropoxycarbonyl)oxy)methyl-4-(2-(1H -imidazol-1-yl)ethoxy)-3-methoxybenzoate

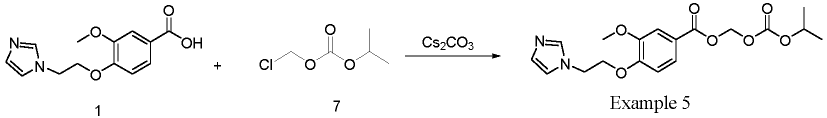

Compound 1 (2.00 g, 7.63 mmol), DMF (25 mL) and cesium carbonate (19.9 g, 61.0 mmol) were added sequentially into a 100 mL single-necked flask and stirred at room temperature for 15 min. To the above reaction solution, a solution of compound 7 (7.56 g, 49.6 mmol) in DMF (5 mL) was added dropwise and the mixture was reacted at room temperature overnight. After the reaction was completed as monitored with TLC (*V*_{dichloromethane}:*V*ₘₑₜₕₐₙₒₗ=20:1), water (50 mL) was added to the reaction solution, and the mixture was extracted with ethyl acetate (50 mL×5). The organic phases were combined and washed with saturated sodium chloride solution (10 mL×3), and the organic phase was collected. The mixture was dried over anhydrous magnesium sulfate, filtered, concentrated and purified by column chromatography (*V*_{dichloromethane}:*V*ₘₑₜₕₐₙₒₗ= 40: 1) to obtain 700 mg of Example 5 compound as a white solid in a yield of 24.2%. ¹H NMR (400 MHz, CDCl₃) *δ* (ppm): 7.69-7.64 (m, 2H, ArH), 7.57 (d, *J*=2.0Hz, 1H, ArH), 7.10-7.06 (m, 2H, ArH), 6.79 (d, *J*=8.4Hz, 1H, ArH), 5.97 (s, 2H, OCH₂O), 4.97-4.90 (m, 1H, CH), 4.39 (t, *J*=4.8Hz, 2H, CH₂), 4.30 (t, *J*=4.8Hz, 2H, CH₂ CH₂), 3.91 (s, 3H, OCH₃), 1.33 (s, 3H, CH₃), 1.31 (s, 3H, CH₃).

### Example 6 Synthesis of Compound 1-acetoxyethyl-4-(2-(1H-imidazol-1-yl)ethoxy)-3-methoxybenzoate

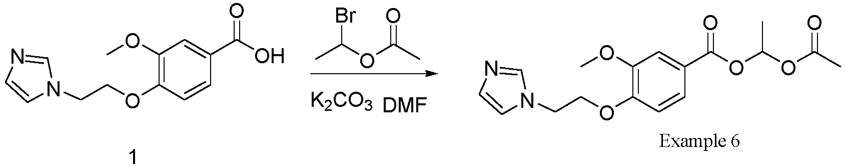

Ethyl 1-bromoacetate (1.25 g, 7.5 mmol), compound 1 (1.31 g, 5 mmol), potassium carbonate (1.04 g, 7.5 mmol) and DMF (30 mL) were added to a 100 mL single-necked flask and stirred at room temperature for 4.5 h under nitrogen atmosphere. The reaction was complete as monitored with TLC (*V*_{dichloromethane}: *V*ₘₑₜₕₐₙₒₗ = 20:1). Water (50 mL) was added to the reaction solution, and the mixture was extracted with ethyl acetate (50 mL×3). The organic phases were combined, washed with saturated brine (60 mL×3), dried over anhydrous sodium sulfate, filtered, concentrated, and purified by flash preparative chromatography (80 g, *V*_{dichloromethane}:*V*ₘₑₜₕₐₙₒₗ=20:1) to obtain 700 mg of Example 6 compound as a white solid with a yield of 40.2%. ¹H NMR (400 MHz, CDCl₃) *δ* (ppm): 7.67 (s, 1H, CH), 7.57 (dd, *J*=8.4 Hz, *J*=2.0 Hz, 1H, ArH), 7.48 (d, *J*=2.0 Hz, 1H, ArH), 7.04-7.00 (m, 3H, ArH), 6.73 (d, *J*=8.8 Hz, 1H, ArH), 4.34-4.32 (m, 2H, NCH₂), 4.24-4.21 (m, 2H, OCH₂), 3.84 (s, 3H, OCH₃), 2.02 (s, 3H, COCH₃), 1.52 (d, *J*=5.6 Hz, 3H, CH₃).

### Example 7 Synthesis of Compound (S)-4-(4-(2-(1H-imidazol-1-yl)ethoxy)-3-methoxybenzamido)phenylalanine hydrochloride

### Step 1

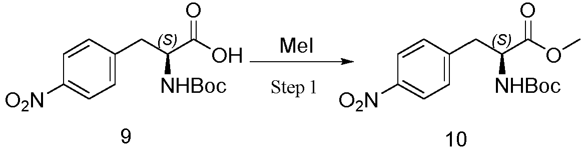

Compound 9, i.e., (S)-2-((tert-butoxycarbonyl)amino)-3-(4-nitrophenyl)propanoic acid (5.00 g, 16.10 mmol), sodium carbonate (8.54 g, 80.1 mmol), iodomethane (11.37 g, 80.1 mmol) and DMF (35 mL) were added sequentially into a 250 mL single-necked flask and stirred at room temperature for 16 h under a nitrogen atmosphere. The reaction of the raw materials was completed as monitored with TLC (*V*_{petroleum ether}:*V*_{ethyl acetate} = 3: 1). The reaction solution was added dropwise into water (200 mL), stirred at room temperature for 2 h and then filtered. The filter cake was washed with water (100 mL × 2). The filter cake was collected and dried in a vacuum drying oven overnight at 45 °C to obtain 5.10 g of intermediate **10** as a light yellow solid with a yield of 98.1%.

¹H NMR (600 MHz, CD₃OD) *δ* (ppm): 8.14 (d, *J*=8.2 Hz, 2H, ArH), 7.30 (d, *J*=8.2 Hz, 2H, ArH), 5.04 (s, 1H, NH), 4.62 (s, 1H, CH), 3.72 (s, 3H, OCH₃), 3.25 (s, 1H, CH₂), 3.11 (s, 1H, CH₂), 1.39 (s, 9H, 3CH₃).

### Step 2

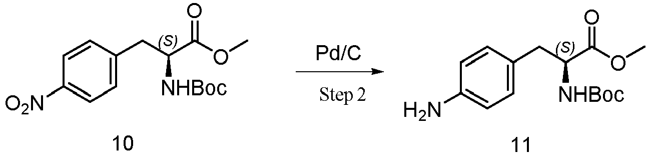

Intermediate 10 (5.10 g, 15.73 mmol), methanol (50 mL), acetic acid (0.3 mL) and 10% Pd/C (770 mg, 15% wt) were added in sequence into a 250 mL single-necked flask, hydrogen was introduced, and the mixture was stirred at room temperature for 2 h under a hydrogen atmosphere (15 psi). The reaction of the starting material was completed as monitored with TLC (*V*_{petroleum ether}: *V*_{dichloromethane}=1:1). The mixture was filtered through celite pad, and the filter cake was rinsed with methanol (100 mL× 2). The filtrate was concentrated to obtain 4.41 g of red oily intermediate 11, which was used directly in the next step without purification.

### Step 3

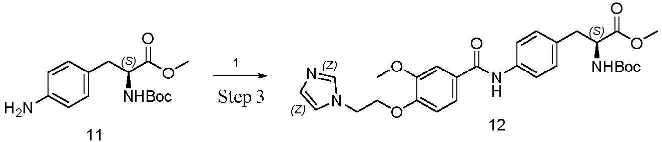

Compound 1 (1.00 g, 3.81 mmol), toluene (15 mL), thionyl chloride (2.3 g, 19.05 mmol) and DMF (0.3 mL) were added to a 100 mL single-necked flask and stirred at 80 °C overnight under a nitrogen atmosphere. The reaction solution was cooled to room temperature and concentrated. It was evaporated into dryness with toluene (30 mL × 3) and then dichloromethane (20 mL) and intermediate 11 (1.35 g, 4.58 mmol) were added. The mixture was stirred at room temperature for 2 h under a nitrogen atmosphere. The reaction of the starting material was completed as monitored with TLC (*V*ₘₑₜₕₐₙₒₗ:*V*_{dichloromethane}=10:1). The mixture was concentrated and purified by column chromatography (*V*_{dichloromethane}: *V*ₘₑₜₕₐₙₒₗ = 25:1) to obtain 2 g of intermediate 12 as a white solid in a yield of 97.4%. ¹H NMR (600 MHz, DMSO- *d*₆) *δ* (ppm): 10.03 (s, 1H, NH), 7.78 (s, 1H, NH), 7.65 (d, *J*=8.5 Hz, 2H, ArH), 7.58 (dd, *J₁*=8.4 Hz, *J₂*= 2.1 Hz, 1H, ArH), 7.54 (d, *J*=2.1 Hz, 1H, ArH), 7.31-7.25 (m, 2H, ArH), 7.19 (d, *J*=8.5 Hz, 2H, ArH), 7.07 (d, *J*=8.5 Hz, 1H), 6.95 (s, 1H, ArH), 4.41 (t, *J*=5.2 Hz, 2H, CH₂), 4.32 (t, *J*=5.2 Hz, 2H, CH₂), 4.17-4.13 (m, 1H, CH), 3.85 (s, 3H, CH₃), 3.61 (s, 3H, CH₃), 2.95 (dd, *J₁*=13.9 Hz, *J₂*=5.3 Hz, 1H, CH₂), 2.83 (dd, *J₁*=13.9 Hz, *J₂=*9.9 Hz, 1H, CH₂), 1.34 (s, 9H, 3CH₃).

### Step 4

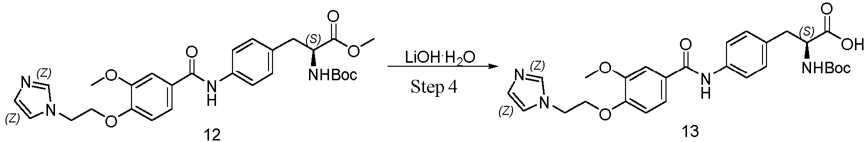

Intermediate 12 (2.00 g, 3.7 mmol), methanol (10 mL), water (2 mL) and LiOH·H₂O (468 mg, 11.10 mmol) were added in sequence into a 50 mL single-necked flask, and stirred at room temperature for 1 h. The reaction of the starting materials was completed as monitored with TLC (*V*_{dichloromethane}:*V*ₘₑₜₕₐₙₒₗ=5: 1). The reaction solution was concentrated, and dilute hydrochloric acid (1N) was added to adjust the pH value to 6. The reaction solution was filtered, and the filter cake was washed with water (20 mL). The filter cake was collected and dried in a drying oven overnight at 45 °C to obtain 1.40 g of intermediate 13 as a white solid with a yield of 85.1%. ¹H NMR (600 MHz, DMSO- *d*₆) *δ* (ppm): 10.02 (s, 1H, NH), 7.71 (s, 1H, NH), 7.64 (d, *J*=8.4 Hz, 2H, ArH), 7.57 (dd, *J₁*=8.4 Hz, *J₂*=2.1 Hz, 1H, ArH), 7.54 (d, *J*=2.1 Hz, 1H, ArH), 7.27 (s, 1H, ArH), 7.20 (d, *J*=8.3 Hz, 2H, ArH), 7.06 (d, *J*=8.4, 2H, ArH), 6.90 (s, 1H, ArH), 4.40 (t, *J*=5.2 Hz, 2H, CH₂), 4.32 (t, *J*=5.2 Hz, 2H, CH₂), 4.09-4.05 (m, 1H, CH), 3.85 (s, 3H, CH₃), 2.98 (dd, *J₁*=13.9 Hz, *J₂=* 5.3 Hz, 1H, CH₂), 2.80 (dd, *J₁*=13.9 Hz, *J₂=* 10.1 Hz, 1H, CH₂), 1.33 (s, 9H, 3CH₃).

### Step 5

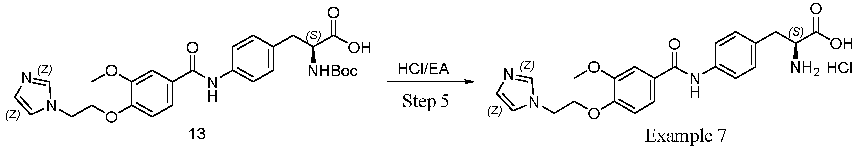

To a 50 mL single-necked flask, were added intermediate 13 (750 mg, 1.43 mmol), ethyl acetate (5 mL) and a solution of HCl in ethyl acetate (10 mL, 4 N) in sequence and stirred at room temperature for 1 h. The reaction of the raw materials was completed *as* monitored with TLC (*V*_{dichloromethane}:*V*ₘₑₜₕₐₙₒₗ = 5:1). The reaction solution was directly filtered, and the filter cake was washed with petroleum ether of low-boiling point (30 mL). The filter cake was collected and dried in a drying oven overnight at 45 °C to obtain 630 mg of Example 7 as a white solid with a yield of 96.0%. ¹H NMR (400 MHz, DMSO- *d*₆) *δ* (ppm): 10.28 (s, 1H, NH), 9.22 (s, 1H, ArH), 8.52 (s, 3H, NH₂HCl), 7.83 (s, 1H, ArH), 7.76 (d, *J*=8.4 Hz, 2H, ArH), 7.71 (s, 1H, ArH), 7.64 (d, *J*=9.9 Hz, 2H, ArH), 7.24 (d, *J*=8.3 Hz, 2H, ArH), 7.11 (d, *J*=8.3 Hz, 1H, ArH), 4.66 (t, *J=5.0* Hz, 2H, CH₂), 4.47 (t, *J=5.0* Hz, 2H, CH₂), 4.11 (d, *J*=6.0 Hz, 1H, CH), 3.85 (s, 3H, CH₃), 3.19-3.08 (m, 2H, CH₂). ¹³C NMR (151 MHz, DMSO- *d*₆) *δ* (ppm): 170.74, 165.22, 150.40, 149.16, 138.96, 136.30, 130.40, 130.13, 128.59, 122.93, 121.68, 121.17, 120.04, 113.74, 112.37, 67.55, 56.57, 53.80, 48.62, 35.61.

### Example 8 Synthesis of Compound (R)-3-(4-(4-(2-(1H-imidazol-1-yl)ethoxy)-3-methoxybenzamido)phenyl)-2-propionic acid hydrochloride

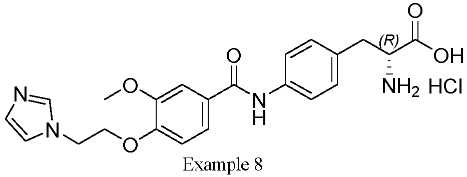

By reference to the synthesis method of Example 7, (S)-2-((tert-butoxycarbonyl)amino)-3-(4-nitrophenyl)propanoic acid was replaced with (R)-2-((tert-butoxycarbonyl)amino)-3-(4-nitrophenyl)propanoic acid to obtain 300 mg of the compound of Example 8 as a white solid with a yield of 85.9%. ¹H NMR (400 MHz, DMSO- *d*₆) *δ* (ppm): 10.27-10.22 (m, 1H, NH), 9.21-9.19 (m, 1H, ArH), 8.50-8.44 (m, 3H, NH₂HCl), 7.82 (s, 1H, ArH), 7.79-7.73 (m, 2H, ArH), 7.71 (s, 1H, ArH), 7.67-7.59 (m, 2H, ArH), 7.27-7.23 (m, 2H, ArH), 7.11 (d, *J*=8.4 Hz, 1H, ArH), 4.65 (t, *J=5.0* Hz, 2H, CH₂), 4.46 (t, *J* =5.1 Hz, 2H, CH₂), 4.12 (s, 1H, CH), 3.85 (s, 3H, CH₃), 3.19-3.08 (m, 2H, CH₂). ¹³C NMR (151 MHz, DMSO- *d*₆) *δ* (ppm): 170.73, 165.23, 150.40, 149.16, 138.96, 136.30, 130.40, 130.13, 128.59, 122.94, 121.69, 121.18, 120.04, 113.76, 112.38, 67.56, 56.58, 53.81, 48.62, 35.61.

### Example 9 Synthesis of Compound (S)-4-(4-(2-(1H-imidazol-1-yl)ethoxy)-3-methoxybenzoyloxy)phenylalanine hydrochloride

### Step 1

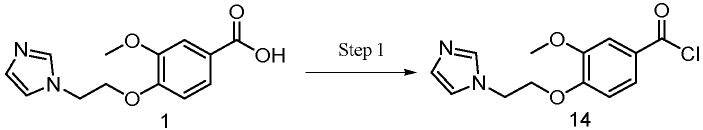

Compound 1 (1.20 g, 4.58 mmol), toluene (20 mL), thionyl chloride (5 mL) and N,N-dimethylformamide (0.1 mL) were added to a 50 mL single-necked flask in sequence. After the addition was completed, the mixture was reacted at 90 °C under nitrogen protection for 2 h. The reaction mixture was concentrated and the residue was evaporated with toluene (20 mL × 3). 1.3 g of intermediate 14 was obtained as a white solid, which was dissolved in acetonitrile (10 mL) for use.

### Step 2

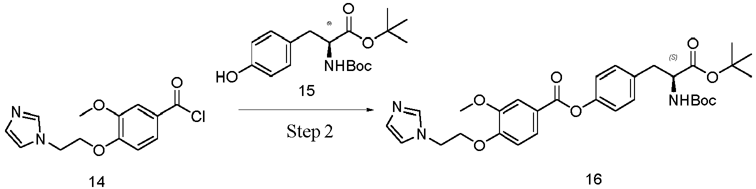

*N*-Boc-L-tyrosine tert-butyl ester (**15**, 929 mg, 2.75 mmol), acetonitrile (20 mL) and triethylamine (463 mg, 4.58 mmol) were added in sequence into a 50 mL single-necked flask. After the addition is completed, the solution of the intermediate 14 in acetonitrile obtained in the first step was added dropwise. After the addition is completed, the reaction was kept at room temperature under nitrogen protection for 2 h. The reaction of the raw material was completed as monitored with TLC (*V*_{dichloromethane}: *V*ₘₑₜₕₐₙₒ = 15:1). Water (50 mL) was added to the reaction solution, and the mixture was stirred for 5 min before separation. The aqueous phase was extracted with dichloromethane (50 mL×3). The organic phases were combined, washed with saturated brine (100 mL), dried over anhydrous sodium sulfate, concentrated and purified by flash preparative chromatography (20 g, *V*_{ethyl acetate}:Vₘₑₜₕₐₙₒₗ = 40:1) to obtain 1.22 g of white foamy solid intermediate 16 in a yield of 92%. ¹H NMR (600 MHz, DMSO- *d*₆) *δ* (ppm): 7.69 (dd, *J₁*=8.4 Hz, *J₂*=2.0 Hz, 1H, ArH), 7.65 (t, *J*=1.1 Hz, 1H, ArH), 7.55 (d, *J*=2.1 Hz, 1H, ArH), 7.27 (d, *J*=8.5 Hz, 2H, ArH), 7.22 (t, *J*=1.2 Hz, 1H, ArH), 7.19-7.08 (m, 4H, ArH, NH), 6.86 (t, *J=*1.1 Hz, 1H, ArH), 4.40-4.35 (m, 2H, NCH₂), 4.34-4.29 (m, 2H, OCH₂), 4.03-3.96 (m, 1H, NCH), 3.81 (s, 3H, OCH₃), 2.93 (dd, *J₁*=13.9 Hz, *J₂*=5.7 Hz, 1H, CH₂), 2.84 (dd, *J₁*=13.8 Hz, *J₂* =9.6 Hz, 1H, CH₂), 1.32 (s, 9H, CH₃), 1.31 (s, 9H, CH₃).

### Step 3

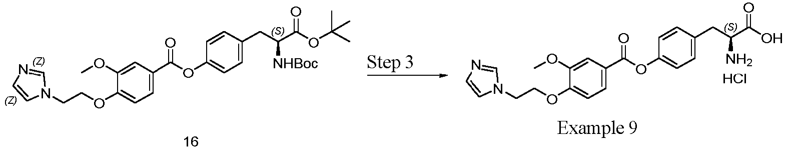

Intermediate 16 (800 mg, 1.38 mmol) and a solution of hydrogen chloride in 1,4-dioxane (2.7 M, 20 mL) were added in sequence into a 50 mL single-necked flask and after the addition is complete, the reaction was kept at 60 °C for 2 h under nitrogen protection. The reaction solution was concentrated, and ethyl acetate (20 mL) was added to the residue and slurried for 2 h. The mixture was filtered and the filter cake was dried under vacuum to obtain 510 mg of the compound of Example 9 as a white solid in a yield of 80%. ¹H NMR (600 MHz, DMSO- *d*₆) *δ* (ppm): 9.17-9.14 (m, 1H, ArH), 8.53 (s, 3H, NH₂, HCl), 7.79-7.76 (m, 1H, ArH), 7.71 (dd, *J₁*=8.4 Hz, *J₂*=2.0 Hz, 1H, ArH), 7.66 (t, *J*=1.7 Hz, 1H, ArH), 7.55 (d, *J*=2.0 Hz, 1H, ArH), 7.33 (d, *J*=8.5 Hz, 2H, ArH), 7.17 (dd, *J₁*=12.2 Hz, *J₂*=8.5 Hz, 3H, ArH), 4.64 (t, *J=5.0* Hz, 2H, NCH₂), 4.47 (t, *J=5.1* Hz, 2H, OCH₂), 4.13 (s, 1H, NH₂CH), 3.80 (s, 3H, OCH₃), 3.17-3.14 (m, 2H, CH₂). ¹³C NMR (151 MHz, DMSO-*d*₆) *δ* (ppm): 170.66, 164.64, 152.40, 150.34, 149.39, 136.32, 133.33, 131.24, 124.42, 122.93, 122.49, 122.40, 120.11, 113.82, 113.20, 67.49, 56.47, 53.72, 48.46, 35.42.

### Example 10 Synthesis of Compound (R)-4-(4-(2-(1H-imidazol-1-yl)ethoxy)-3-methoxybenzoyloxy)phenylalanine hydrochloride

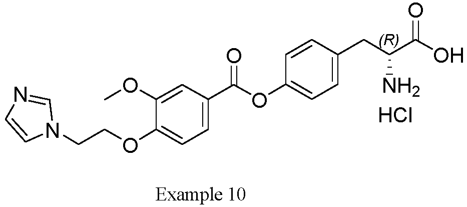

By reference to the synthesis method of Example 9, *N*-Boc-L-tyrosine tert-butyl ester was replaced with *N*-Boc-D-tyrosine tert-butyl ester to obtain 650 mg of the compound of Example 10 as a white solid in a yield of 96.3%. ¹H NMR (600 MHz, DMSO-*d*₆) *δ* (ppm): 9.18 (s, 1H, COOH), 8.54 (s, 3H, NH₂, ArH), 7.81 (t, *J*=1.8 Hz, 1H, ArH), 7.76-7.74 (m, 1H, ArH), 7.70 (t, *J*=1.8 Hz, 1H, ArH), 7.59 (d, *J*=2.4 Hz, 1H, ArH), 7.39-7.35 (m, 2H, ArH), 7.25-7.17 (m, 3H, ArH), 4.68 -4.67 (m, 2H, CH₂), 4.54-4.49 (m, 2H, CH₂), 4.17 (t, *J*=6.6 Hz, 1H), 3.84 (s, 3H, CH₃), 3.19-3.18 (m, 2H, CH₂); ¹³C NMR (151 MHz, DMSO-*d*₆) *δ* (ppm): 170.72, 164.64, 152.40, 150.35, 149.40, 136.36, 133.29, 131.23, 124.42, 122.91, 122.50, 122.42, 120.18, 113.80, 113.21, 67.46, 56.47, 53.69, 48.45, 35.47.

### Example 11 Biological Activity Test

### Study on the antithrombotic activities of the compounds of the present invention in vivo in rats

104 SD rats (purchased from Weitong Lihua Experimental Animal Technology Co., Ltd., certificate number: 20211103Aazz0619000910), male, weighing 180-220 g, were randomly divided into 13 groups according to body weight after 7 days of adaptive feeding, with 8 rats in each group, namely, normal control group, compound A group, compound A methyl ester group, and Example 1-10 compound groups respectively. Each group was given corresponding drugs by gavage (dosage was 15 mg/kg), and the normal control group was given an equal volume of solvent, all of which were given by single gavage. One hour after the last administration, the rats were anesthetized with 3% chloral hydrate intraperitoneally, and the right common carotid artery and left external jugular vein were isolated in a supine position. A polyethylene tube was taken with an inner diameter of 1.5 mm and a length of 10 cm, a 6 cm long silk thread (preweighed) was inserted into it, and the polyethylene tube was filled with normal saline solution of heparin (50 U/ml). One end of the polyethylene tube was inserted into the left external jugular vein, heparin (50 U/kg) was injected, and the other end was inserted into the right common carotid artery. After blood flow was opened for 15 minutes, the blood flow was interrupted and the silk thread was quickly taken out and weighed. The total weight minus the original silk thread weight was the wet weight of the thrombus. The silk thread was placed in an oven for drying at 70°C. The dry weight of the thrombus was obtained by weighing the silk thread minus the original silk thread weight, and the inhibition rate was calculated. Wet weight inhibition rate (%) = (wet weight of thrombus in normal control group - wet weight of thrombus in drug-treated group) / wet weight of thrombus in normal control group × 100%. Dry weight inhibition rate (%) = (dry weight of thrombus in normal control group - dry weight of thrombus in drug-treated group) / dry weight of thrombus in normal control group × 100%.

The experimental results are shown in Table 1. Compared with the blank control group, each drug-treated group can significantly reduce the wet weight and dry weight of thrombus (P<0.01), and has a significant inhibitory effect on thrombosis; the antithrombotic effect of compound A is equivalent to that of its methyl ester, with no significant difference; compared with compound A and compound A methyl ester, the compounds of the present invention has a significantly stronger effect on reducing the wet weight and dry weight of thrombus, with inhibition rates of thrombus wet weights being more than 45%, and inhibition rates of thrombus dry weights being more than 50%.

**Table 1. Effects of the compounds of the present invention on the dry and wet weights of arteriovenous bypass thrombi in rats ( x̅ ±s, n=8)**

| Groups | Dosage (mg/kg) | Thrombus wet weight (mg) | Inhibition rate (%) | Thrombus dry weight (mg) | Inhibition rate (%) |
|---|---|---|---|---|---|
| Blank control | - | 101.1±17.7 | - | 34.0±7.5 | - |
| Compound A | 15 | 69.8+11.2^{★★} | 31.03 | 21.8±6.0^{★★} | 36.03 |
| Compound A methyl ester | 15 | 71.3±8.7^{★★} | 29.42 | 23.0±8.2^{★★} | 32.35 |
| Example 1 | 15 | 54.8±12.7^{★★*##} | 45.86 | 16.0±3.4^{★★*##} | 52.94 |
| Example 2 | 15 | 52.3±10.5^{★★**##} | 48.3 | 15.4±5.0^{★★*##} | 54.78 |
| Example 3 | 15 | 47.3±6.3^{★★**##} | 53.3 | 12.3±5.7^{★★**##} | 63.97 |
| Example 4 | 15 | 53.0±14.9^{★★**##} | 47.6 | 15.3±3.7^{★★*##} | 55.15 |
| Example 5 | 15 | 47.0±8.7^{★★**##} | 53.5 | 15.0±4.5^{★★*##} | 55.88 |
| Example 6 | 15 | 45.1±8.6^{★★**##} | 55.4 | 14.3±5.0^{★★**##} | 58.09 |
| Example 7 | 15 | 54.4±9.8^{★★*##} | 46.2 | 16.1±3.3^{★★*##} | 52.57 |
| Example 8 | 15 | 52.0±13.5^{★★**##} | 48.6 | 14.6±3.8^{★★**##} | 56.99 |
| Example 9 | 15 | 51.1±12.7^{★★**##} | 49.4 | 14.5±6.2^{★★**##} | 57.35 |
| Example 10 | 15 | 53.1±11.8^{★★**##} | 47.5 | 15.3±2.8^{★★*##} | 55.14 |

| | | | | | |
|---|---|---|---|---|---|
| *P<0.05 ^{★★}P<0.01 vs blank; * P < 0.05, ** P < 0.01 vs compound A; ^{#} P<0.05, ^{##} P<0.01 vs Compound A methyl ester | | | | | |

### Study on the pharmacokinetics of the compounds of the present invention in vivo in rats

Eighteen SD male rats (purchased from Weitong Lihua Experimental Animal Technology Co., Ltd., certificate number: 20211103Aazz0619000910), weighing 240-280 g, were randomly divided into 6 groups, with 3 rats in each group. The animals were fasted overnight before administration. On the day of administration, the compounds of Examples 3, 4, 5, 6, Compound A, and Compound A methyl ester were gavaged to the animals in each groups respectively, and the dosage was 15 mg/kg (calculated as Compound A). Before administration and 0.25, 0.5, 1, 2, 4, 6, 8, and 24 h after administration, 0.15 mL of blood was collected from the animals through the jugular sinus and placed in an EDTA-K₂ anticoagulant tube containing 3 µL of dichlorvos aqueous solution (10 mg/mL). After centrifugation, plasma was collected, and was placed in the sample, and then 10 µL of plasma sample was taken, 500 µL of acetonitrile containing IS (verapamil 5 ng·mL⁻¹, glibenclamide 50 ng·mL⁻¹, tolbutamide 200 ng·mL⁻¹, and diclofenac 200 ng·mL⁻¹) was added, vortexed for 10 min, and then centrifuged at 3700 rpm for 10 min, and then 70 µL of supernatant and 70 µL of water were added, and vortexed for 10 min. An aliquot of 2 µL of the mixture was injected into the LC-MS/MS system (Triple Quad 5500+: LC-MS-MS-022) to measure blood concentration of the compounds. WinNonlin 8.0 software was used to analyze the pharmacokinetic parameters. The analysis results are shown in Table 2.

**Table 2. Pharmacokinetic parameters**

| Pharmacoki netic parameters | Example 3 | Example 4 | Example 5 | Example 6 | Compound A | Compound A methyl ester |
|---|---|---|---|---|---|---|
| | p.o.-15 mg/kg | p.o.-15 mg/kg | p.o.-15 mg/kg | p.o.-15 mg/kg | p.o.-15 mg/kg | p.o.-15 mg/kg |
| | mean value | mean value | mean value | mean value | mean value | mean value |
| AUC₀₋ₜ (ng·h·mL⁻¹) | 3230 | 2740 | 2890 | 2850 | 1612 | 1486 |
| AUC_{0-inf} (ng h mL) | 3375 | 2910 | 3000 | 3170 | 1725 | 1605 |
| Cₘₐₓ (ng·mL⁻¹) | 8590 | 4470 | 7080 | 8140 | 604 | 513 |
| Tₘₐₓ (h) | 0.250 | 0.250 | 0.250 | 0.250 | 0.350 | 0.460 |
| T_{1/2} (h) | 3.06 | 5.75 | 3.43 | 4.86 | 10.72 | 8.4 |
| MRTo-t (h) | 0.47 | 0.91 | 0.85 | 1.09 | 5.4 | 6.5 |
| F^{a)} | 200.37% | 169.97% | 179.28% | 176.8% | / | 92.18% |

| | | | | | | |
|---|---|---|---|---|---|---|
| Note: a) is the bioavailability relative to compound A. | | | | | | |

The experimental results show that area under the concentration-time curve (AUC) of the compounds of Examples 3, 4, 5, and 6 of the present invention are significantly improved compared with Compound A and the methyl ester of Compound A, and the relative bioavailability is 200.37%, 169.97%, 179.28%, and 176.8%, respectively, which is significantly higher than the methyl ester of Compound A. In addition, after gavage administration, the peak concentration (Cₘₐₓ) was significantly higher than that of compound A and the methyl ester of compound A, and the time to peak (Tₘₐₓ) was significantly shortened. It is suggested that compared with compound A and the methyl ester of compound A, the compounds of the present invention have significantly higher bioavailability, faster onset of action and stronger effect.

All references mentioned herein are incorporated by reference. It should be understood that the technical solutions of the present invention may be changed and modified without departing from the spirit and scope of the present disclosure.

## Claims

1. A compound having a structure represented by the following formula (I), 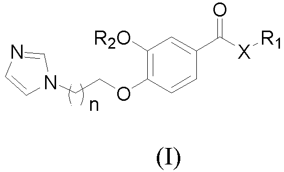 or a pharmaceutically acceptable salt, stereoisomer, tautomer, prodrug, or solvate thereof, wherein:
X is selected from O or NH;
R₁ is selected from C₃ - C₁₂ alkyl, C₃ - C₁₂ alkenyl, C₃ - C₁₂ alkynyl, C₃ - C₁₂ cycloalkyl, and C₆ - C₁₄ aryl, wherein the alkyl, alkenyl, alkynyl, and cycloalkyl are optionally substituted by halogen, cyano, C₁ - C₁₂ alkoxy, C₁ - C₁₂ alkoxycarbonyl, C₁ - C₁₂ alkoxycarbonyloxy, C₁ - C₁₂ alkylacyloxy, carboxyl, C₁ - C₁₂ alkylthio, C₁ - C₁₂ alkylthiocarbonyl, C₁ - C₁₂ alkylthiocarbonyloxy, C₁ - C₁₂ alkylthiocarbonylthio, C₁ - C₁₂ alkylacylthio, C₁ - C₁₂ alkylamino, C₁ - C₁₂ alkylaminocarbonyl, C₁ - C₁₂ alkylaminoacyloxy, C₁ - C₁₂ alkylamide, or C₁ - C₁₂ alkylaminoamide; the aryl is optionally substituted by C₁ - C₁₂ alkyl, C₁ - C₁₂ alkoxy, OH, halogen, cyano, nitro, or carboxyl, wherein the alkyl and the alkoxy are optionally substituted by OH, amino, carboxyl, nitro, cyano, halogen, C₁ - C₁₂ alkoxy, or C₃ - C₁₂ cycloalkyl ; or R₁ is selected from C₁ - C₂ alkyl substituted by C₁ - C₁₂ alkoxycarbonyloxy or C₁ - C₁₂ alkylacyloxy, or benzyl substituted by hydroxyl;
R₂ is selected from H or C₁ - C₁₂ alkyl; and
n is selected from 0, 1, 2, or 3.

2. The compound or pharmaceutically acceptable salt, stereoisomer, tautomer, prodrug, or solvate thereof according to claim 1, wherein:
R₁ is selected from C₃ - C₁₂ alkyl, C₃ - C₁₂ cycloalkyl, or C₆ - C₁₄ aryl, wherein the alkyl and cycloalkyl are optionally substituted by halogen, cyano, C₁ - C₁₂ alkoxy, C₁ - C₁₂ alkoxycarbonyl, C₁ - C₁₂ alkoxycarbonyloxy, C₁ - C₁₂ alkylacyloxy, carboxyl, C₁ - C₁₂ alkylthio, C₁ - C₁₂ alkylthiocarbonyl, C₁ - C₁₂ alkylthiocarbonyloxy, C₁ - C₁₂ alkylthiocarbonylthio, C₁ - C₁₂ alkylacylthio, C₁ - C₁₂ alkylamino, C₁ - C₁₂ alkylaminocarbonyl, C₁ - C₁₂ alkylaminoacyloxy, C₁ - C₁₂ alkylamide, or C₁ - C₁₂ alkylaminoamide; the aryl is optionally substituted by C₁ - C₁₂ alkyl, C₁ - C₁₂ alkoxy, OH, halogen, cyano, nitro, or carboxyl, wherein the alkyl and the alkoxy are optionally substituted by OH, amino, carboxyl, nitro, cyano, halogen, C₁ - C₁₂ alkoxy, or C₃ - C₁₂ cycloalkyl ; or R₁ is selected from C₁ - C₂ alkyl substituted by C₁ - C₁₂ alkoxycarbonyloxy or C₁ - C₁₂ alkylacyloxy , or benzyl substituted by hydroxyl.

3. The compound or pharmaceutically acceptable salt, stereoisomer, tautomer, prodrug, or solvate thereof according to claim 1 or 2, wherein:
R₁ is selected from C₃ - C₁₂ alkyl, C₃ - C₁₂ cycloalkyl, or C₆ - C₁₄ aryl, wherein the alkyl and cycloalkyl are optionally substituted by halogen, C₁ - C₁₂ alkoxy, C₁ - C₁₂ alkoxycarbonyl, C₁ - C₁₂ alkoxycarbonyloxy, C₁ - C₁₂ alkylacyloxy, or carboxyl; the aryl is optionally substituted by C₁ - C₁₂ alkyl, C₁ - C₁₂ alkoxy, or OH, wherein the alkyl and alkoxy are optionally substituted by OH, amino, or carboxyl; or R₁ is selected from C₁ - C₂ alkyl substituted by C₁ - C₁₂ alkoxycarbonyloxy or C₁ - C₁₂ alkylacyloxy, or benzyl substituted by hydroxyl.

4. The compound or pharmaceutically acceptable salt, stereoisomer, tautomer, prodrug, or solvate thereof according to any one of the preceding claims, wherein:
R₁ is selected from C₃ - C₆ alkyl, C₃ - C₈ cycloalkyl, or phenyl, wherein the alkyl and cycloalkyl are optionally substituted by halogen, C₁ - C₆ alkoxy, C₁ - C₆ alkoxycarbonyl, C₁ -C₆ alkoxycarbonyloxy, C₁ -C₆ alkylacyloxy, or carboxyl; the phenyl is optionally substituted by C₁ - C₆ alkyl, C₁ - C₆ alkoxy, or OH, wherein the alkyl and alkoxy are optionally substituted by amino or carboxyl; or R₁ is selected from C₁ - C₂ alkyl substituted by C₁ - C₆ alkoxycarbonyloxy or C₁ - C₆ alkylacyloxy, or benzyl substituted by hydroxyl.

5. The compound or pharmaceutically acceptable salt, stereoisomer, tautomer, prodrug, or solvate thereof according to any one of the preceding claims, wherein:
R₁ is selected from: n-propyl, isopropyl,

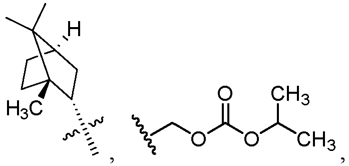

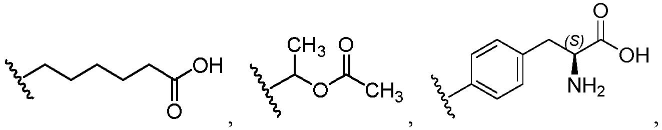

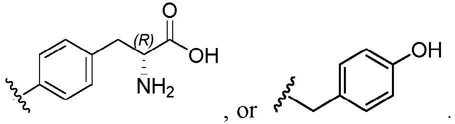

6. The compound according to claim 1, wherein the compound is selected from: 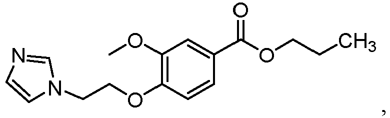 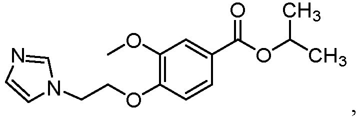 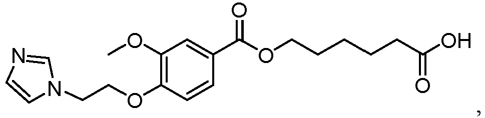 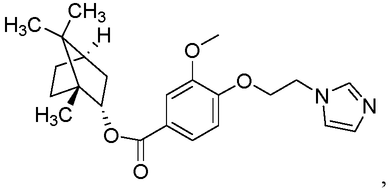 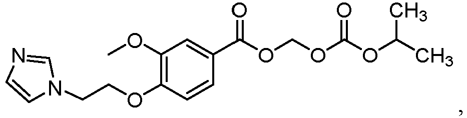 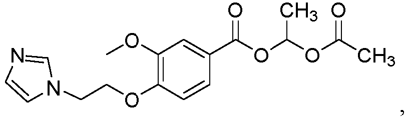 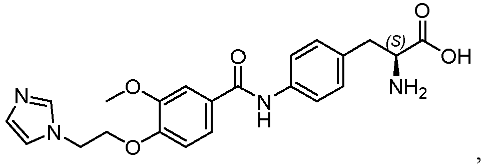 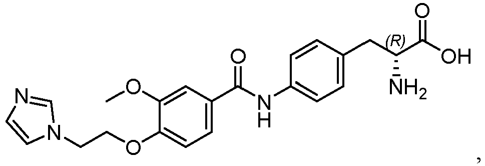 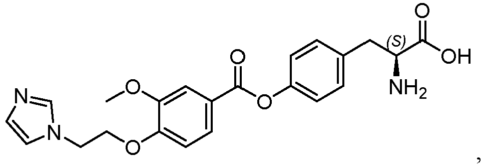 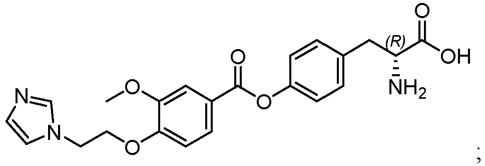 or a pharmaceutically acceptable salt, stereoisomer, tautomer, prodrug, or solvate thereof.

7. A pharmaceutical composition comprising the compound or pharmaceutically acceptable salt, stereoisomer, tautomer, prodrug, or solvate thereof according to any one of claims 1 to 6, and a pharmaceutically acceptable carrier.

8. Use of the compound according to any one of claims 1 to 6 in the manufacture of a medicament for treating and/or preventing thromboembolic diseases.

9. Use of the compound according to any one of claims 1 to 6 in the manufacture of a medicament for treating and/or preventing a disease caused by platelet aggregation.

10. The use according to claim 8 or 9, wherein the disease is selected from atherosclerosis, coronary heart disease, myocardial infarction, ischemic stroke, peripheral vascular disease, multiple sclerosis, scleroderma, Raynaud's phenomenon caused by multiple sclerosis, deep vein thrombosis, lower extremity vein thrombosis, and intermittent claudication.
